# EUROPEAN PATENT APPLICATION

(11) **EP 3 741 750 A1**
(43) Date of publication of application: **25.11.2020**
(21) Application number: 19382396.0
(22) Date of filing: 20.05.2019
(51) Int. Cl.: C07D 403/04, A61K 31/4192, A61P 29/00

(54) **4-(1H-1,2,3-TRIAZOL-1-YL)PYRROLIDINE-2-CARBOXYLIC ACID DERIVATIVES HAVING ACTIVITY AGAINST PAIN**

(71) Applicant: ESTEVE PHARMACEUTICALS, S.A., 08038 Barcelona (ES)
(72) Inventor: Díaz-Fernández, Jose-Luís, 08242 Manresa (ES); Almansa-Rosales, Carmen, 08022 Barcelona (ES); Pericàs-Brondo, Miquel-Àngel, 08950 Esplugues de Llobregat (ES); Alza-Barrios, Esther, 43003 Tarragona (ES)
(74) Representative: Ruiz, Nicolas Vincent

(57) **Abstract**

The present invention relates to 4-(1*H*-1,2,3-triazolyl-1-yl)pyrrolidine-2-carboxylic acid derivatives (I) having pharmacological activity towards the α₂δ subunit of the voltage-gated calcium channel, to processes for the preparation of such compounds, to pharmaceutical compositions comprising them, and to their use in therapy, in particular for the treatment of pain.

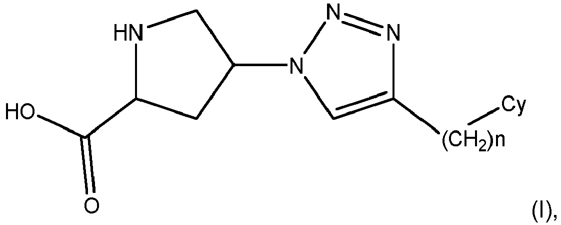

## Description

### FIELD OF THE INVENTION

The present invention relates to compounds having pharmacological activity towards the α₂δ subunit of the voltage-gated calcium channel, and more particularly to 4-(1*H-*1,2,3-triazolyl-1-yl)pyrrolidine-2-carboxylic acid derivatives having this pharmacological activity, to processes for the preparation of such compounds, to pharmaceutical compositions comprising them, and to their use in therapy, in particular for the treatment of pain.

### BACKGROUND OF THE INVENTION

The adequate management of pain constitutes an important challenge, since currently available treatments provide in many cases only modest improvements, leaving many patients unrelieved (Turk, D.C., Wilson, H.D., Cahana, A.; 2011; Lancet; 377; 2226-2235). Pain affects a big portion of the population with an estimated prevalence of 20 % and its incidence, particularly in the case of chronic pain, is increasing due to the population ageing. Additionally, pain is clearly related to comorbidities, such as depression, anxiety and insomnia, which leads to important productivity losses and socio-economical burden (Goldberg, D.S., McGee, S.J.; 2011; BMC Public Health; 11; 770).

Voltage-gated calcium channels (VGCC) are required for many key functions in the body. Different subtypes of voltage-gated calcium channels have been described (Zamponi et al., Pharmacol Rev. 2015 67:821-70). The VGCC are assembled through interactions of different subunits, namely α₁(Caᵥα₁), β(Caᵥβ) α₂δ(Caᵥα₂δ) and γ (Caᵥγ). The α₁ subunits are the key porous forming units of the channel complex, being responsible for the Ca²⁺ conduction and generation of Ca²⁺ influx. The α₂δ, β, and γ subunits are auxiliary, although very important for the regulation of the channel, since they increase the expression of the α₁ subunits in the plasma membrane as well as modulate their function, resulting in functional diversity in different cell types. Based on their physiological and pharmacological properties, VGCC can be subdivided into low voltage-activated T-type (Caᵥ3.1, Caᵥ3.2, and Caᵥ3.3), and high voltage-activated L-(Caᵥ1.1 through Caᵥ1.4), N-(Caᵥ2.2), P/Q-(Caᵥ2.1), and R-(Caᵥ2.3) types, depending on the channel forming Caᵥα subunits. All of these five subclasses are found in the central and peripheral nervous systems. Regulation of intracellular calcium through activation of these VGCC plays obligatory roles in: 1) neurotransmitter release, 2) membrane depolarization and hyperpolarization, 3) enzyme activation and inactivation, and 4) gene regulation (Perret and Luo, Neurotherapeutics. 2009 6:679-92; Zamponi et al., 2015 *supra;* Neumaier et al., Prog Neurobiol. 2015 129:1-36.). A large body of data has clearly indicated that VGCC are implicated in mediating various disease states including pain processing. Drugs interacting with the different calcium channel subtypes and subunits have been developed. Current therapeutic agents include drugs targeting L-type Caᵥ1.2 calcium channels, particularly 1,4-dihydropyridines, which are widely used in the treatment of hypertension. T-type (Caᵥ3) channels are the target of ethosuximide, widely used in absence epilepsy. Ziconotide, a peptide blocker of N-type (Caᵥ2.2) calcium channels, has been approved as a treatment of intractable pain. (Perret and Luo, 2009, *supra;* Vink and Alewood, Br J Pharmacol. 2012 167:970-89.).

The Caᵥ1 and Caᵥ2 subfamilies contain an auxiliary α₂δ subunit, which is the therapeutic target of the gabapentinoid drugs of value in certain epilepsies and chronic neuropathic pain. To date, there are four known α₂δ subunits, each encoded by a unique gene and all possessing splice variants. Each α₂δ protein is encoded by a single messenger RNA and is post-translationally cleaved and then linked by disulfide bonds. Four genes encoding α₂δ subunits have now been cloned. α₂δ-1 was initially cloned from skeletal muscle and shows a fairly ubiquitous distribution. The α₂δ-2 and α₂δ-3 subunits were subsequently cloned from brain. The most recently identified subunit, α₂δ-4, is largely non-neuronal. The human α₂δ-4 protein sequence shares 30, 32 and 61% identity with the human α₂δ-1, α₂δ-2 and α₂δ-3 subunits, respectively. The gene structure of all α₂δ subunits is similar. All α₂δ subunits show several splice variants (Davies et al., Trends Pharmacol Sci. 2007 28:220-8.; Dolphin AC, Nat Rev Neurosci. 2012 13:542-55., Biochim Biophys Acta. 2013 1828:1541-9.).

The Caᵥα₂δ-1 subunit may play an important role in neuropathic pain development (Perret and Luo, 2009, *supra;* Vink and Alewood, 2012, *supra*). Biochemical data have indicated a significant Caᵥα₂δ-1, but not Caᵥα₂δ-2, subunit upregulation in the spinal dorsal horn, and DRG (dorsal root ganglia) after nerve injury that correlates with neuropathic pain development. In addition, blocking axonal transport of injury-induced DRG Caᵥα₂δ-1 subunit to the central presynaptic terminals diminishes tactile allodynia in nerve injured animals, suggesting that elevated DRG Caᵥα₂δ-1 subunit contributes to neuropathic allodynia.

The Caᵥα₂δ-1 subunit (and the Caᵥα₂δ-2, but not Caᵥα₂δ-3 and Caᵥα₂δ-4, subunits) is the binding site for gabapentin which has anti-allodynic/ hyperalgesic properties in patients and animal models. Because injury-induced Caᵥα₂δ-1 expression correlates with neuropathic pain development and maintenance, and various calcium channels are known to contribute to spinal synaptic neurotransmission and DRG neuron excitability, injury-induced Caᵥα₂δ-1 subunit upregulation may contribute to the initiation and maintenance of neuropathic pain by altering the properties and/or distribution of VGCC in the subpopulation of DRG neurons and their central terminals, therefore modulating excitability and/or synaptic neuroplasticity in the dorsal horn. Intrathecal antisense oligonucleotides against the Caᵥα₂δ-1 subunit can block nerve injury-induced Caᵥα₂δ-1 upregulation and prevent the onset of allodynia and reserve established allodynia.

As mentioned above, the α₂δ subunits of VGCC form the binding site for gabapentin and pregabalin, which are structural derivatives of the inhibitory neurotransmitter GABA although they do not bind to GABAA, GABAB, or benzodiazepine receptors, or alter GABA regulation in animal brain preparations. The binding of gabapentin and pregabalin to the Caᵥα₂δ subunit results in a reduction in the calcium-dependent release of multiple neurotransmitters, leading to efficacy and tolerability for neuropathic pain management. Gabapentinoids may also reduce excitability by inhibiting synaptogenesis (Perret and Luo, 2009, *supra;* Vink and Alewood, 2012, *supra,* Zamponi et al., 2015, *supra*).

Existing pain therapies include non-steroidal anti-inflammatory drugs (NSAIDs), opioid agonists, and antidepressants, but they are much less than optimal regarding their safety ratio. All of them show limited efficacy and a range of secondary effects difficulting their use, especially in chronic settings.

Accordingly, there is still a need to find compounds that have an alternative or improved pharmacological activity in the treatment of pain, being both effective and showing the desired selectivity, and having good "drugability" properties, i.e. good pharmaceutical properties related to administration, distribution, metabolism and excretion. Compounds of the present invention that show pharmacological activity towards the α₂δ subunit, in particular the α₂δ-1 subunit, of the voltage-gated calcium channel, result in an innovative, effective and alternative solution for the treatment of pain.

In view of the existing results of the currently available therapies and clinical practices, the present invention offers a solution relevant for the treatment of pain. This was mainly achieved by providing the compounds according to the invention that bind to the α₂δ subunit, in particular the α₂δ-1 subunit, of the voltage-gated calcium channel.

### SUMMARY OF THE INVENTION

The present invention discloses novel compounds with high affinity towards the α₂δ subunit of the voltage-gated calcium channel, as well as their use for the treatment of pain. More particularly those compounds are 4-(1*H*-1,2,3-triazolyl-1-yl)pyrrolidine-2-carboxylic acid derivatives.

As this invention is aimed at providing compounds which act as ligands of the α₂δ subunit, in particular the α₂δ-1 subunit, of the voltage-gated calcium channel, it is a very preferred embodiment if the compound has a binding expressed as Kᵢ responding to the following scales:
Ki(α₂δ-1) is preferably < 10000 nM, more preferably < 5000 nM, even more preferably < 500 nM or even more preferably < 100 nM.

In a main aspect, the present invention is directed to novel triazolyl pyrrolidine derivatives of general formula (I): 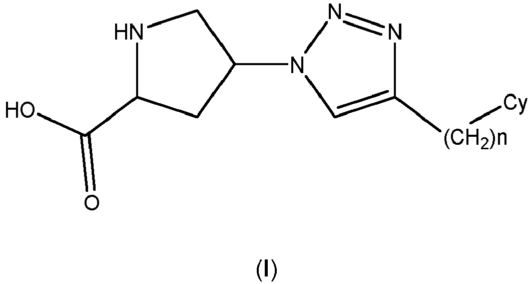 wherein Cy represents a group selected from: 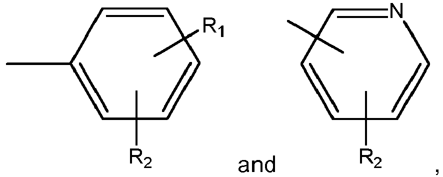 and wherein R₁, R₂ and n are as defined below.

Another object of the invention relates to the different processes for preparation of compounds of general formula (I).

Another object of the invention refers to such compounds of general formula (I) for use as a medicament and more particularly for use in the treatment or prophylaxis of pain or pain related conditions, especially neuropathic pain, inflammatory pain or other pain conditions involving allodynia and/or hyperalgesia.

It is also an object of the invention to provide pharmaceutical compositions comprising one or more compounds of general formula (I) with at least one pharmaceutically acceptable excipient. The pharmaceutical compositions in accordance with the invention can be adapted in order to be administered by any route of administration, be it orally or parenterally, such as pulmonarily, nasally, rectally and/or intravenously. Therefore, the formulation in accordance with the invention may be adapted for topical or systemic application, particularly for dermal, subcutaneous, intramuscular, intra-articular, intraperitoneal, pulmonary, buccal, sublingual, nasal, percutaneous, vaginal, oral or parenteral application.

### DETAILED DESCRIPTION OF THE INVENTION

In its main aspect, the present invention relates to compounds of general formula (I): 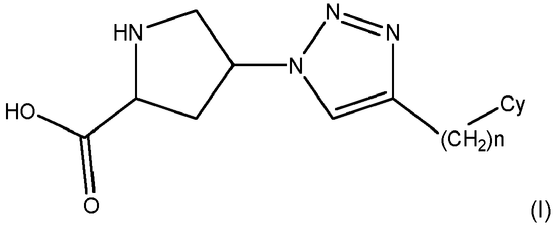 wherein
Cy represents a group selected from: 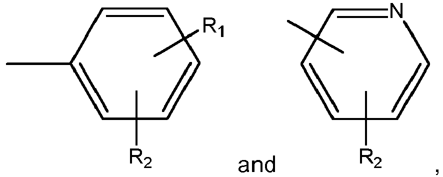 n is 0 or 1;
R₁ is selected from the group consisting of hydrogen, halogen, -CN, linear or branched substituted or unsubstituted C₁₋₆ alkyl, linear or branched substituted or unsubstituted C₂₋₆ alkenyl, and linear or branched substituted or unsubstituted C₁₋₆ alkoxy;
R₂ is selected from the group consisting of hydrogen, halogen, linear or branched substituted or unsubstituted C₁₋₆ alkyl, linear or branched substituted or unsubstituted C₂₋₆ alkenyl, linear or branched substituted or unsubstituted C₁₋₆ alkoxy and -(CH₂)ₚ-NRₐR_{b},
   p is 0, 1, 2, or 3;
   Rₐ and R_{b} are independently selected from hydrogen, linear or branched C₁₋₆ alkyl and substituted or unsubstituted benzyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding physiologically acceptable salt or solvate thereof.

In a more specific aspect of the present invention, the following compounds are excluded from formula (I):
- 4-(4-(3-chlorophenyl)-1*H*-1,2,3-triazol-1-yl)pyrrolidine-2-carboxylic acid,
- 4-(4-(2-methoxyphenyl)-1*H*-1,2,3-triazol-1-yl)pyrrolidine-2-carboxylic acid,
- 4-(4-(3-methoxyphenyl)-1*H*-1,2,3-triazol-1-yl)pyrrolidine-2-carboxylic acid,
- 4-(4-(4-fluorophenyl)-1*H*-1,2,3-triazol-1-yl)pyrrolidine-2-carboxylic acid,
- 4-(4-(4-methoxyphenyl)-1*H*-1,2,3-triazol-1-yl)pyrrolidine-2-carboxylic acid,
- 4-(4-(pyridin-2-yl)-1*H*-1,2,3-triazol-1-yl)pyrrolidine-2-carboxylic acid,
- 4-(4-phenyl-1*H*-1,2,3-triazol-1-yl)pyrrolidine-2-carboxylic acid,
- 4-(4-(2-chloro)-1*H*-1,2,3-triazol-1-yl)pyrrolidine-2-carboxylic acid,
- 4-(4-(3,5-difluorophenyl)-1*H*-1,2,3-triazol-1-yl)pyrrolidine-2-carboxylic acid.

"Halogen" as referred in the present invention represent fluorine, chlorine, bromine or iodine, preferably fluorine, chlorine or bromine, and especially fluorine or chlorine.

In the context of this invention, C₁₋₆ alkyl is understood as meaning saturated, linear or branched hydrocarbons. It encompasses e.g. -CH₃ and -CH₂-CH₃. In these radicals, C₁₋₂-alkyl represents C1- or C2-alkyl, C₁₋₃-alkyl represents C1-, C2- or C3-alkyl, C₁₋₄-alkyl represents C1-, C2-, C3- or C4-alkyl, C₁₋₅-alkyl represents C1-, C2-, C3-, C4-, or C5-alkyl, C₁₋₆-alkyl represents C1-, C2-, C3-, C4-, C5- or C6-alkyl. The C₁₋₆ alkyl radicals are preferably methyl, ethyl, propyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, if substituted also CHF₂, CF₃ or CH₂OH etc. Preferably C₁₋₆ alkyl is understood in the context of this invention as methyl, ethyl, propyl, butyl, pentyl, or hexyl; more preferably it is a C₁₋₄ alkyl like methyl, ethyl, propyl or butyl.

C₂₋₆alkenyl is understood as meaning unsaturated, linear or branched hydrocarbons. It encompasses groups like e.g. -CH=CH-CH₃. The C₂₋₆ alkenyl radicals are preferably vinyl (ethenyl), allyl (2-propenyl). Preferably in the context of this invention C₂₋₆-alkenyl is ethylene, propylene, butylene, pentylene, or hexylene; or it is a C₂₋₄-alkenyl, like ethylene, propylene, or butylenes.

A C₁₋₆ alkoxy group, as referred to in the present invention, is undersood as meaning a -O-C₁₋₆ alkyl group wherein C₁₋₆ alkyl is as defined previously.

In the context of this invention, benzyl means a -CH₂-phenyl group.

In connection with C₁₋₆ alkyl, C₂₋₆ alkenyl, C₁₋₆ alkoxy and benzyl - unless defined otherwise - the term substituted in the context of this invention is understood as meaning replacement of at least one hydrogen radical on a carbon atom by halogen (F, Cl, Br, I), a linear or branched C₁₋₆ alkyl group, -CF₃, -CH₂F, -CHF₂, -CN, -OH, -SH, -NH₂, oxo, -(C=O)R', -OR', -SR', -SOR', -SO₂R', -NHR', -NR'R" whereby R' and optionally R" for each substitutent independently represents a linear or branched C₁₋₆-alkyl radical.

In a particularly preferred embodiment of the present invention, the C₁₋₆ alkyl, C₂₋₆ alkenyl, C₁₋₆ alkoxy and benzyl groups are unsubstituted or substituted by one or more susbstituents selected from a C₁₋₆alkyl radical, F, Cl, I, Br, OH and -OR' whereby R' for each substitutent independently represents a linear or branched C₁₋₆-alkyl radical.

In a further more preferred embodiment of the present invention, the C₁₋₆ alkyl, C₂₋₆ alkenyl and C₁₋₆ alkoxy groups are unsubstituted.

In another more preferred embodiment of the present invention, the benzyl groups are unsubstituted.

More than one replacement on the same molecule and also on the same carbon atom is possible with the same or different substituents. This includes for example 3 hydrogens being replaced on the same C atom, as in the case of CF₃, or at different places of the same molecule, as in the case of e.g. -CH(OH)-CH=CH-CHCl₂.

When several R' radicals are present simultaneously in Formula I they may be identical or different.

The term "salt" is to be understood as meaning any form of the active compound according to the invention in which this assumes an ionic form or is charged and is coupled with a counter-ion (a cation or anion) or is in solution. By this are also to be understood complexes of the active compound with other molecules and ions, in particular complexes which are formed via ionic interactions.

The term "physiologically acceptable salt" means in the context of this invention any salt that is physiologically tolerated (most of the time meaning not being toxic-especially not caused by the counter-ion) if used appropriately for a treatment especially if used on or applied to humans and/or mammals.

These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH₄, but in particular (mono)- or (di)sodium, (mono)- or (di)potassium, magnesium or calcium salts. Physiologically acceptable salts can also be formed with anions or acids and in the context of this invention is understood as meaning salts of at least one of the compounds used according to the invention as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

Any compound that is a solvate of a compound according to the invention like a compound according to general formula I defined above is understood to be also covered by the scope of the invention. Methods of solvation are generally known within the art. Suitable solvates are pharmaceutically acceptable solvates. The term "solvate" according to this invention is to be understood as meaning any form of the active compound according to the invention in which this compound has attached to it via noncovalent binding another molecule (most likely a polar solvent). Especially preferred examples include hydrates and alcoholates, like methanolates or ethanolates.

Any compound that is a prodrug of a compound according to the invention like a compound according to general formula I defined above is understood to be also covered by the scope of the invention. The term "prodrug" is used in its broadest sense and encompasses those derivatives that are converted *in vivo* to the compounds of the invention. Such derivatives would readily occur to those skilled in the art, and include, depending on the functional groups present in the molecule and without limitation, the following derivatives of the present compounds: esters, amino acid esters, phosphate esters, metal salts sulfonate esters, carbamates, and amides. Examples of well known methods of producing a prodrug of a given acting compound are known to those skilled in the art and can be found e.g. in Krogsgaard-Larsen et al. "Textbook of Drug design and Discovery" Taylor & Francis (April 2002).

Any compound that is a N-oxide of a compound according to the invention like a compound according to general formula I defined above is understood to be also covered by the scope of the invention.

The compounds of formula (I) as well as their salts or solvates of the compounds are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts. This applies also to its solvates or prodrugs.

The obtained reaction products may, if desired, be purified by conventional methods, such as crystallisation and chromatography. Where the above described processes for the preparation of compounds of the invention give rise to mixtures of stereoisomers, these isomers may be separated by conventional techniques such as preparative chromatography. If there are chiral centers the compounds may be prepared in racemic form, or individual enantiomers may be prepared either by enantiospecific synthesis or by resolution.

In a first particular embodiment of the compounds of formula (I) according to the invention, Cy represents a group:

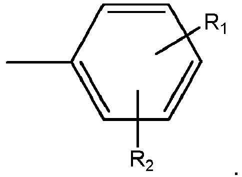

In a preferred aspect of this first embodiment, the compounds of formula (I) are those where R₁ represents a hydrogen, a halogen, more preferably a Br, Cl, or F, a -CN, an unsubstituted C₁₋₆ alkyl, preferably a methyl (-CH₃), or a unsubstituted C₁₋₆ alkoxy, preferably a methoxy (-O-CH₃).

In another preferred aspect of this first embodiment, the compounds of formula (I) are those where R₂ represents a hydrogen, a halogen, more preferably a Br, Cl, or F, -(CH₂)ₚ-NRₐR_{b}, wherein p is 1, Rₐ is a hydrogen or an unsubstituted C₁₋₆ alkyl, preferably a methyl (-CH₃), and R_{b} is a hydrogen, an unsubstituted C₁₋₆ alkyl, preferably a methyl (-CH₃), or an unsubstituted benzyl.

In a further preferred aspect of this first embodiment, Cy represents a group: 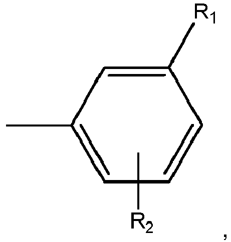 where R₁ is in position 3 of the phenyl and R₁ and R₂ have the same meaning than previously.

In a second particular embodiment of the compounds of formula (I) according to the invention, Cy represents a group:

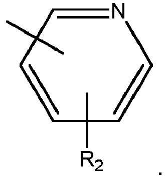

In a preferred aspect of this second embodiment, the compounds of formula (I) according to the invention are those where R₂ represents a hydrogen or a halogen, more preferably a Cl.

In a further preferred aspect of this second embodiment, the compounds of formula (I) according to the invention are those where Cy represents a group: 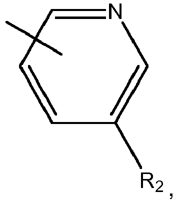 where R₂ as the same meaning than previously.

In a more preferred variant of the invention, the compounds of formula (I) are selected from the group consisiting of:
- (2*S*,4*S*)-4-(4-(3-fluorophenyl)-1*H*-1,2,3-triazol-1-yl)pyrrolidine-2-carboxylic acid,
- (2*S*,4*S*)-4-(4-(3-chlorophenyl)-1*H*-1,2,3-triazol-1-yl)pyrrolidine-2-carboxylic acid,
- (2*S*,4*S*)-4-(4-(2-methoxyphenyl)-1*H*-1,2,3-triazol-1-yl)pyrrolidine-2-carboxylic acid,
- (2*S*,4*S*)-4-(4-(3-methoxyphenyl)-1*H*-1,2,3-triazol-1-yl)pyrrolidine-2-carboxylic acid,
- (2*S*,4*S*)-4-(4-(4-fluorophenyl)-1*H*-1,2,3-triazol-1-yl)pyrrolidine-2-carboxylic acid,
- (2*S*,4*S*)-4-(4-(4-methoxyphenyl)-1*H*-1,2,3-triazol-1-yl)pyrrolidine-2-carboxylic acid,
- (2*S*,4*S*)-4-(4-phenyl-1*H*-1,2,3-triazol-1-yl)pyrrolidine-2-carboxylic acid,
- (2*S*,4*S*)-4-(4-(*m*-tolyl)-1*H*-1,2,3-triazol-1-yl)pyrrolidine-2-carboxylic acid,
- (2*S*,4*S*)-4-(4-(pyridin-2-yl)-1*H*-1,2,3-triazol-1-yl)pyrrolidine-2-carboxylic acid,
- (2*S*,4*S*)-4-(4-(3,4-difluorophenyl)-1*H*-1,2,3-triazol-1-yl)pyrrolidine-2-carboxylic acid,
- (2*S*,4*S*)-4-(4-(2-chloro-4-methoxyphenyl)-1*H*-1,2,3-triazol-1-yl)pyrrolidine-2-carboxylic acid,
- (2*S*,4*S*)-4-(4-(2-chlorophenyl)-1*H*-1,2,3-triazol-1-yl)pyrrolidine-2-carboxylic acid,
- (2*S*,4*S*)-4-(4-(2,4-dichlorophenyl)-1*H*-1,2,3-triazol-1-yl)pyrrolidine-2-carboxylic acid,
- (2*S*,4*S*)-4-(4-(3,4-dichlorophenyl)-1*H*-1,2,3-triazol-1-yl)pyrrolidine-2-carboxylic acid,
- (2*S*,4*S*)-4-(4-(3,5-difluorophenyl)-1*H*-1,2,3-triazol-1-yl)pyrrolidine-2-carboxylic acid,
- (2*S*,4*S*)-4-(4-benzyl-1*H*-1,2,3-triazol-1-yl)pyrrolidine-2-carboxylic acid,
- (2*S*,4*S*)-4-(4-(4-bromophenyl)-1*H*-1,2,3-triazol-1-yl)pyrrolidine-2-carboxylic acid,
- (2*S*,4*S*)-4-(4-(pyridin-3-yl)-1*H*-1,2,3-triazol-1-yl)pyrrolidine-2-carboxylic acid,
- (2*S*,4*S*)-4-(4-(pyridin-4-yl)-1*H*-1,2,3-triazol-1-yl)pyrrolidine-2-carboxylic acid,
- (2*S*,4*S*)-4-(4-(5-chloropyridin-3-yl)-1*H*-1,2,3-triazol-1-yl)pyrrolidine-2-carboxylic acid,
- (2*S*,4*S*)-4-(4-(3-cyanophenyl)-1*H*-1,2,3-triazol-1-yl)pyrrolidine-2-carboxylic acid,
- (2*S*,4*S*)-4-(4-(4-cyanophenyl)-1*H*-1,2,3-triazol-1-yl)pyrrolidine-2-carboxylic acid,
- (2*S*,4*S*)-4-(4-(3-chlorobenzyl)-1*H*-1,2,3-triazol-1-yl)pyrrolidine-2-carboxylic acid,
- (2*S*,4*S*)-4-(4-(3-bromophenyl)-1*H*-1,2,3-triazol-1-yl)pyrrolidine-2-carboxylic acid,
- (2*S*,4*S*)-4-(4-(3-((dimethylamino)methyl)-5-fluorophenyl)-1*H*-1,2,3-triazol-1 - yl)pyrrolidine-2-carboxylic acid,
- (2*S*,4*S*)-4-(4-(3-fluoro-5-((methylamino)methyl)phenyl)-1*H*-1,2,3-triazol-1 - yl)pyrrolidine-2-carboxylic acid,
- (2*S*,4*S*)-4-(4-(3,5-dichlorophenyl)-1*H*-1,2,3-triazol-1-yl)pyrrolidine-2-carboxylic acid,
- (2*S*,4*S*)-4-(4-(3-((benzyl(methyl)amino)methyl)-5-fluorophenyl)-1*H*-1,2,3-triazol-1-yl)pyrrolidine-2-carboxylic acid,
- (2*S*,4*S*)-4-(4-(3-((benzylamino)methyl)-5-fluorophenyl)-1*H*-1,2,3-triazol-1 - yl)pyrrolidine-2-carboxylic acid,
- (2*S*,4*S*)-4-(4-(4-chlorobenzyl)-1*H*-1,2,3-triazol-1-yl)pyrrolidine-2-carboxylic acid,
or a physiologically acceptable salt or solvate thereof.

In a further more preferred variant of the invention, the compounds of formula (I) are selected from the group consisiting of:
- (2*S*,4*S*)-4-(4-(3-fluorophenyl)-1*H*-1,2,3-triazol-1-yl)pyrrolidine-2-carboxylic acid,
- (2*S*,4*S*)-4-(4-(*m*-tolyl)-1*H*-1,2,3-triazol-1-yl)pyrrolidine-2-carboxylic acid,
- (2*S*,4*S*)-4-(4-(3,4-difluorophenyl)-1*H*-1,2,3-triazol-1-yl)pyrrolidine-2-carboxylic acid,
- (2*S*,4*S*)-4-(4-(2-chloro-4-methoxyphenyl)-1*H*-1,2,3-triazol-1-yl)pyrrolidine-2-carboxylic acid,
- (2*S*,4*S*)-4-(4-(2,4-dichlorophenyl)-1*H*-1,2,3-triazol-1-yl)pyrrolidine-2-carboxylic acid,
- (2*S*,4*S*)-4-(4-(3,4-dichlorophenyl)-1*H*-1,2,3-triazol-1-yl)pyrrolidine-2-carboxylic acid,
- (2*S*,4*S*)-4-(4-benzyl-1*H*-1,2,3-triazol-1-yl)pyrrolidine-2-carboxylic acid,
- (2*S*,4*S*)-4-(4-(4-bromophenyl)-1*H*-1,2,3-triazol-1-yl)pyrrolidine-2-carboxylic acid,
- (2*S*,4*S*)-4-(4-(pyridin-3-yl)-1*H*-1,2,3-triazol-1-yl)pyrrolidine-2-carboxylic acid,
- (2*S*,4*S*)-4-(4-(pyridin-4-yl)-1*H*-1,2,3-triazol-1-yl)pyrrolidine-2-carboxylic acid,
- (2*S*,4*S*)-4-(4-(5-chloropyridin-3-yl)-1*H*-1,2,3-triazol-1-yl)pyrrolidine-2-carboxylic acid,
- (2*S*,4*S*)-4-(4-(3-cyanophenyl)-1*H*-1,2,3-triazol-1-yl)pyrrolidine-2-carboxylic acid,
- (2*S*,4*S*)-4-(4-(4-cyanophenyl)-1*H*-1,2,3-triazol-1-yl)pyrrolidine-2-carboxylic acid,
- (2*S*,4*S*)-4-(4-(3-chlorobenzyl)-1*H*-1,2,3-triazol-1-yl)pyrrolidine-2-carboxylic acid,
- (2*S*,4*S*)-4-(4-(3-bromophenyl)-1*H*-1,2,3-triazol-1-yl)pyrrolidine-2-carboxylic acid,
- (2*S*,4*S*)-4-(4-(3-((dimethylamino)methyl)-5-fluorophenyl)-1*H*-1,2,3-triazol-1-yl)pyrrolidine-2-carboxylic acid,
- (2*S*,4*S*)-4-(4-(3-fluoro-5-((methylamino)methyl)phenyl)-1*H*-1,2,3-triazol-1-yl)pyrrolidine-2-carboxylic acid,
- (2*S*,4*S*)-4-(4-(3,5-dichlorophenyl)-1*H*-1,2,3-triazol-1-yl)pyrrolidine-2-carboxylic acid,
- (2*S*,4*S*)-4-(4-(3-((benzyl(methyl)amino)methyl)-5-fluorophenyl)-1*H*-1,2,3-triazol-1-yl)pyrrolidine-2-carboxylic acid,
- (2*S*,4*S*)-4-(4-(3-((benzylamino)methyl)-5-fluorophenyl)-1*H*-1,2,3-triazol-1 - yl)pyrrolidine-2-carboxylic acid,
- (2*S*,4*S*)-4-(4-(4-chlorobenzyl)-1*H*-1,2,3-triazol-1-yl)pyrrolidine-2-carboxylic acid,
or a physiologically acceptable salt or solvate thereof.

Any compound referred to herein is intended to represent such specific compound as well as certain variations or forms. In particular, compounds referred to herein may have asymmetric centers and therefore exist in different enantiomeric or diastereomeric forms. Thus, any given compound referred to herein is intended to represent any one of a racemate, one or more enantiomeric forms, one or more diastereomeric forms, and mixtures thereof. Likewise, stereoisomerism or geometric isomerism about the double bond is also possible, therefore in some cases the molecule could exist as (E)-isomer or (Z)-isomer (*trans* and *cis* isomers). If the molecule contains several double bonds, each double bond will have its own stereoisomerism, that could be the same as, or different to, the stereoisomerism of the other double bonds of the molecule. Furthermore, compounds referred to herein may exist as atropisomers. All the stereoisomers including enantiomers, diastereoisomers, geometric isomers and atropisomers of the compounds referred to herein, and mixtures thereof, are considered within the scope of the present invention.

Furthermore, any compound referred to herein may exist as tautomers. Specifically, the term tautomer refers to one of two or more structural isomers of a compound that exist in equilibrium and are readily converted from one isomeric form to another. Common tautomeric pairs are amine-imine, amide-imidic acid, keto-enol, lactam-lactim, etc.

In another aspect, the invention refers to processes for obtaining the compounds of general formula (I).

Compounds of formula (I) as defined above are prepared as shown in Scheme 1: 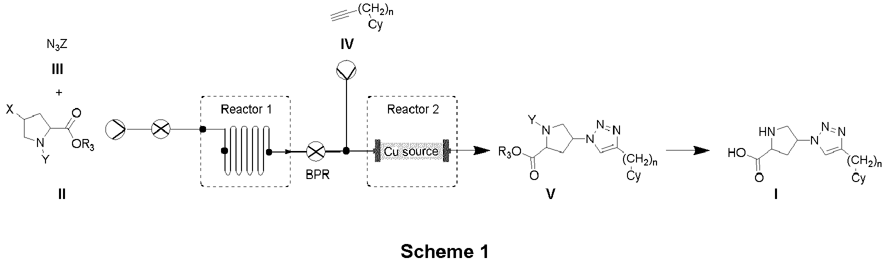 wherein Cy has the meaning as defined above for a compound of formula (I), X represents a leaving group (such as chloro, bromo, iodo, mesylate, tosylate, nosylate or triflate), R₃ represents an C₁₋₆ alkyl group (such as tert-butyl), Y represents a protecting group of the amino function, such as Boc (*tert*-butoxycarbonyl) or Teoc (2-(trimethylsilyl)ethoxycarbonyl) and N₃Z represents a suitable azide derivative, such as sodium azide, potassium azide, tosyl azide or trimethylsilyl azide.

The first step is a continuous flow process that provides a compound of formula (V) starting from a stock solution of a compound of formula (II) with an azide reagent of formula (III), preferably sodium azide, in organic solvent, preferably tetrahydrofuran, dimethylformamide, dioxane or dimethyl sulfoxide or mixtures of these organic solvents with water. The solution is pumped continuously through reactor 1 at a temperature range of 50-200 °C, preferably 130 °C and pressure range 0-750 psi, preferably 75 psi. The outcome of this reaction is then mixed continuously with a stock solution of a compound of formula (IV) in an organic solvent, preferably tetrahydrofuran, dimethylformamide, 1,4-dioxane or dimethyl sulfoxide or mixtures of these organic solvents with water, which is pumped through reactor 2 at a temperature range between 0-300 °C, preferably at room temperature and a pressure range between 0-1000 psi, preferably 75 psi. Reactor 2 can include a copper source such as copper (I) iodide, copper (I) bromide, copper (I) chloride, copper (II) sulfate, copper (II) acetate or copper metal. These copper sources may be immobilized or impregnated or heterogenized onto different supports, such as nanoparticles, silica, alumina or polymeric resins, preferably Amberlyst A-21 resin. Additionaly, an additive such as sodium ascorbate, potassium ascorbate, a base such as potassium carbonate, N,N-diisopropylethylamine or triethylamine or a small organic molecule, such as proline, can be used.

The second step is the deprotection of the amino group from compounds of formula (V). Boc or Teoc deprotection can be effected by any suitable method, such as treatment with an acid, preferably HCI or trifluoroacetic acid in an appropriate solvent such as 1,4-dioxane, dichloromethane, ethyl acetate or a mixture of an organic solvent and water. Additionaly an additive such as triethylsilane can be used. These acidic conditions are also able to convert a compound in which R₃ is *tert*-butyl to a compound of formula (I) in which R₃ is hydrogen. Alternatively, the deprotection reaction can be carried out by treatment with ZnBr₂ in an organic solvent, preferably dichloromethane; alternatively, for Teoc deprotection, by reaction with CsF in an organic solvent, preferably dimethylformamide at a temperature range of 20-130 °C, optionally under microwaves irradiation.

The compounds of general formula (II), (III) and (IV) are commercially available or can be prepared by conventional methods described in the bibliography.

Moreover, certain compounds of the present invention can also be obtained starting from other compounds of formula (I) by appropriate conversion reactions of functional groups, in one or several steps, using well-known reactions in organic chemistry under standard experimental conditions.

In addition, a compound of formula (I) can be obtained in an enantiopure form from an enantiopure compound of formula (II) or by resolution of a racemic compound of formula (I) either by chiral preparative HPLC of by crystallization of a diastereomeric salt. Alternatively, the resolution step can be carried out at a previous stage, using any suitable intermediate.

Another aspect of the invention refers to a pharmaceutical composition which comprises at least a compound according to the invention as described above according to general formula (I) or a pharmaceutically acceptable salt or steroisomer thereof, and at least a pharmaceutically acceptable carrier, adjuvant or vehicle. The present invention thus provides pharmaceutical compositions comprising at least a compound of this invention, or a pharmaceutically acceptable salt or stereoisomers thereof together with at least a pharmaceutically acceptable carrier, adjuvant, or vehicle, for administration to a patient.

Examples of pharmaceutical compositions include any solid (of tablets, dragees, capsules, granules, pills, chewing gums, powders, drops, gels etc.) or liquid (juices, syrups, solutions and suspensions) composition for oral, topical or parenteral administration.

In a preferred embodiment the pharmaceutical compositions are in oral form, either solid or liquid. Suitable dose forms for oral administration may be tablets, capsules, syrops or solutions and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the apropriate unit dosage form. Adequate excipients can be used, such as bulking agents, buffering agents or surfactants.

The compounds of the invention can be formulated as deposits in dissolved form or in patches, for percutaneous application.

Skin applications include ointments, gels, creams, lotions, suspensions or emulsions.

The mentioned formulations will be prepared using standard methods such as those described or referred to in the Spanish and US Pharmacopoeias and similar reference texts.

Administration of the compounds or compositions of the present invention may be by any suitable method, such as intravenous infusion, oral preparations, and intraperitoneal and intravenous administration. Oral administration is preferred because of the convenience for the patient and the chronic character of the diseases to be treated.

Generally an effective administered amount of a compound of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.

Another aspect of the invention refers to the use of a compound of formula (I) according to the invention or a pharmaceutically acceptable salt or isomer thereof in the manufacture of a medicament.

Another aspect of the invention refers to a compound of formula (I) according to the invention as described above, or a pharmaceutically acceptable salt or isomer thereof, for use as a medicament.

A further aspect of the invention refers to a compound of formula (I) according to the invention as described above, or a pharmaceutically acceptable salt or isomer thereof, for use in the treatment of pain. Preferably the pain is medium to severe pain, visceral pain, chronic pain, cancer pain, migraine, inflammatory pain, acute pain or neuropathic pain, allodynia or hyperalgesia. This may include mechanical allodynia or thermal hyperalgesia.

Another aspect of the invention refers to the use of a compound of formula (I) according to the invention in the manufacture of a medicament for the treatment or prophylaxis of pain.

In a preferred embodiment the pain is selected from medium to severe pain, visceral pain, chronic pain, cancer pain, migraine, inflammatory pain, acute pain or neuropathic pain, allodynia or hyperalgesia, also preferably including mechanical allodynia or thermal hyperalgesia.

Another aspect of this invention relates to a method of treating or preventing pain which method comprises administering to a patient in need of such a treatment a therapeutically effective amount of at least a compound of formula (I) according to the invention as above defined or a pharmaceutical composition thereof. Among the pain syndromes that can be treated are medium to severe pain, visceral pain, chronic pain, cancer pain, migraine, inflammatory pain, acute pain or neuropathic pain, allodynia or hyperalgesia, whereas this could also include mechanical allodynia or thermal hyperalgesia.

The present invention is illustrated below with the aid of examples. These illustrations are given solely by way of example and do not limit the general spirit of the present invention.

### General Experimental Part

### SYNTHESIS DESCRIPTION

### EXAMPLES

The following abbreviations are used:
ACN: Acetonitrile
Anh: Anhydrous
Aq: Aqueous
CH: Cyclohexane
DCM: Dichloromethane
DIAD: Diisopropyl azodicarboxylate
DIBAL: Diisobutylaluminium hydride
DIPEA: *N,N*-Diisopropylethylamine
DMA: *N,N*-Dimethylacetamide
EtOAc: Ethyl acetate
EtOH: Ethanol
Ex: Example
h: Hour/s
HPLC: High-performance liquid chromatography
MeOH: Methanol
MS: Mass spectrometry
Min: Minutes
PPh₃: Triphenylphosphine
Ret: Retention time
rt: Room temperature
Sat: Saturated
TBAF: Tetrabutylammonium fluoride
TBAI: Tetrabutylammonium iodide
TFA: Trifluoroacetic acid
THF: Tetrahydrofuran

The following methods were used to generate the HPLC-MS data:
Method A: Column Luna PFP 4.6x100mm, 5 µm; flow rate 1 mL/min; A: H₂O; B: MeOH; Gradient: 10% to 100% B in 10 min, isocratic 100% B 5 min.
Method B: Column Luna PFP 4.6x100mm, 5 µm; flow rate 1 mL/min; A: H₂O (0.1% Formic Acid); B: MeOH; Gradient: 10% to 100% B in 10 min, isocratic 100% B 5 min.
Method C: Zorbax Eclipse C8 4.6x150mm, 5 µm; flow rate 1 mL/min; A: H₂O (0.1% Formic Acid); B: MeOH; Gradient: 10% to 100% B in 10 min, isocratic 100% B 5 min.
Method D: Column BEH C18 2.1 x 50 mm, 1.7 µm, flow rate 0.61 mL/min; A: NH₄HCO₃ 10 mM, B: ACN, C: MeOH + 0.1% formic acid; gradient 0.3 min 98% A, 98% A to 0:95:5 A:B:C in 2.7 min; 0:95:5 A:B:C to 100 % B in 0.1 min; isocratic 2 min 100% B.

### Example 1: (2S,4S)-4-(4-(3-Fluorophenyl)-1H-1,2,3-triazol-1-yl)pyrrolidine-2-carboxylic acid.

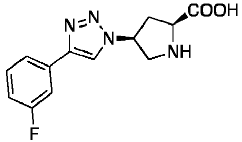

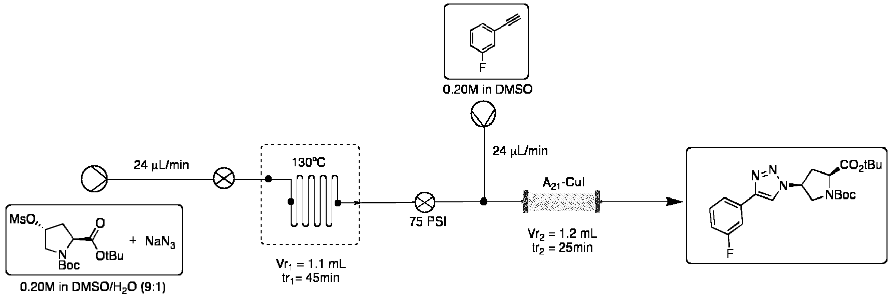

**a) di-*tert*-Butyl (2*S*,4*S*)-4-(4-(3-fluorophenyl)-1*H*-1,2,3-triazol-1-yl)pyrrolidine-1,2-dicarboxylate.** A stock solution of 0.2 M concentration with respect to both di-*tert*-butyl (2S,4R)-4-((methylsulfonyl)oxy)pyrrolidine-1,2-dicarboxylate and sodium azide (1.0 equiv) was prepared by dissolution in 9:1 DMSO/H₂O. The solution was pumped continuously at a flow rate of 24 µL·min⁻¹ through a 1.1 mL mixing chip at 130 °C and 75 psi. The outcome of this reaction was then mixed continuously with a 0.2 M stock solution of 1-ethynyl-3-fluorobenzene (1.0 equiv), which was pumped at a flow rate of 24 µL·min⁻¹ through a 1.2 mL packed bed column filled with Amberlyst A-21-Cul resin (Cu functionalization of 1.27 mmol·g⁻¹ of resin), at room temperature. After 2.5 h, the collected volume was quenched with NH₄Cl. The aqueous layer was extracted with EtOAc (x3). The organic layers were combined and washed with H₂O (x3). Finally, the organic layers were collected, dried over Na₂SO₄, filtered and concentrated in vacuo. The crude product was purified by flash chromatography on silica gel (cyclohexane/EtOAc 7:3) to afford the title compound (135 mg, 52% yield).
**b) (2*S*,4*S*)-4-(4-(3-Fluorophenyl)-1*H*-1,2,3-triazol-1-yl)pyrrolidine-2-carboxylic acid.** To a solution of the compound obtained in step a) (100 mg, 0.231 mmol) in dichloromethane (1 mL) trifluoracetic acid (9 mL, 13 mmol, 9:1 ratio DCM:TFA) and triethylsilane (0.6 µL, 5.8 µmοl) were added. The resulting mixture was stirred at room temperature for 16 h. The solvent was evaporated under reduce pressure and the crude mixture was purified by Dowex 50WX8 (water:ammonia (up to 30%)) to afford the title compound (29.6 mg, 46% yield).

HPLC (Method B): Retention time 5.945 min; ESI⁺-MS *m*/*z,* 277.1 (M+H).

This method was used for the preparation of Ex 2-30 using suitable starting materials:

| **EX** | **Structure** | **Chemical name** | **Meth od** | **Ret (min)** | **MS** |
|---|---|---|---|---|---|
| 2 | 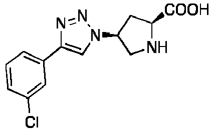 | (2*S*,4*S*)-4-(4-(3-chlorophenyl)-1*H*-1,2,3-triazol-1-yl)pyrrolidine-2-carboxylic acid | A | 6.799 | 293.1 (M+H) |
| 3 | 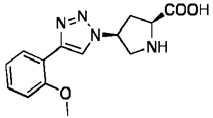 | (2*S*,4*S*)-4-(4-(2-methoxyphenyl)-1*H-*1,2,3-triazol-1-yl)pyrrolidine-2-carboxylic acid | D | 1.00 | 289.0 |
| 4 | 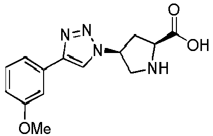 | (2*S*,4*S*)-4-(4-(3-methoxyphenyl)-1*H-*1,2,3-triazol-1-yl)pyrrolidine-2-carboxylic acid | B | 5.866 | 289.1 |
| 5 | 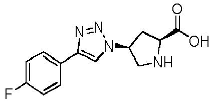 | (2*S*,4*S*)-4-(4-(4-fluorophenyl)-1 *H*-1,2,3-triazol-1-yl)pyrrolidine-2-carboxylic acid | D | 0.97 | 279.9 |
| 6 | 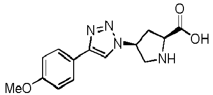 | (2*S*,4*S*)-4-(4-(4-methoxyphenyl)-1*H-*1,2,3-triazol-1-yl)pyrrolidine-2-carboxylic acid | D | 0.94 | 289.0 |
| 7 | 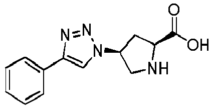 | (2*S*,4*S*)-4-(4-phenyl-1*H*-1,2,3-triazol-1-yl)pyrrolidine-2-carboxylic acid | B | 5.145 | 259.1 |
| 8 | 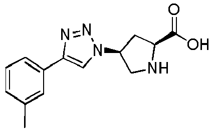 | (2*S*,4*S*)-4-(4-(*m*-tolyl)-1*H*-1,2,3-triazol-1-yl)pyrrolidine-2-carboxylic acid | B | 6.339 | 273.1 |
| 9 | 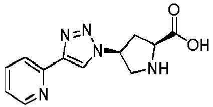 | (2*S*,4*S*)-4-(4-(pyridin-2-yl)-1*H*-1,2,3-triazol-1-yl)pyrrolidine-2-carboxylic acid | D | 0.71 | 260.0 |
| 10 | 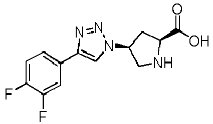 | (2*S*,4*S*)-4-(4-(3,4-difluorophenyl)-1*H-*1,2,3-triazol-1-yl)pyrrolidine-2-carboxylic acid | B | 6.897 | 295.1 |
| 11 | 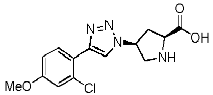 | (2*S*,4*S*)-4-(4-(2-chloro-4-methoxyphenyl)-1*H-*1,2,3-triazol-1-yl)pyrrolidine-2-carboxylic acid | A | 6.555 | 323.1 |
| 12 | 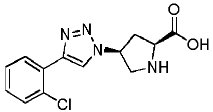 | (2*S*,4*S*)-4-(4-(2-chlorophenyl)-1 *H*-1,2,3-triazol-1-yl)pyrrolidine-2-carboxylic acid | B | 5.974 | 293.1 |
| 13 | 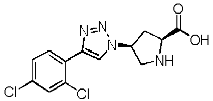 | (2*S*,4*S*)-4-(4-(2,4-dichlorophenyl)-1*H-*1,2,3-triazol-1-yl)pyrrolidine-2-carboxylic acid | B | 7.521 | 327.0 |
| 14 | 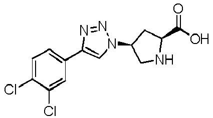 | (2*S*,4*S*)-4-(4-(3,4-dichlorophenyl)-1*H-*1,2,3-triazol-1-yl)pyrrolidine-2-carboxylic acid | B | 8.251 | 327.0 |
| 15 | 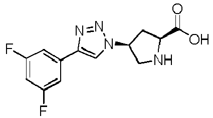 | (2*S*,4*S*)-4-(4-(3,5-difluorophenyl)-1*H-*1,2,3-triazol-1-yl)pyrrolidine-2-carboxylic acid | A | 6.117 | 295.1 |
| 16 | 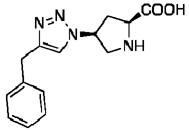 | (2*S*,4*S*)-4-(4-benzyl-1*H*-1,2,3-triazol-1-yl)pyrrolidine-2-carboxylic acid | B | 4.221 | 273.1 |
| 17 | 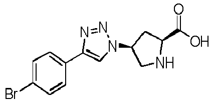 | (2*S*,4*S*)-4-(4-(4-bromophenyl)-1 *H*-1,2,3-triazol-1-yl)pyrrolidine-2-carboxylic acid | D | 1.24 | 337.0 |
| 18 | 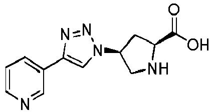 | (2*S*,4*S*)-4-(4-(pyridin-3-yl)-1*H*-1,2,3-triazol-1-yl)pyrrolidine-2-carboxylic acid | A | 3.772 | 260.1 |
| 19 | 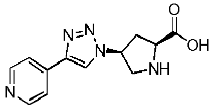 | (2*S*,4*S*)-4-(4-(pyridin-4-yl)-1*H*-1,2,3-triazol-1-yl)pyrrolidine-2-carboxylic acid | A | 3.895 | 260.1 |
| 20 | 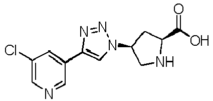 | (2*S*,4*S*)-4-(4-(5-chloropyridin-3-yl)-1*H-*1,2,3-triazol-1-yl)pyrrolidine-2-carboxylic acid | A | 4.991 | 294.1 |
| 21 | 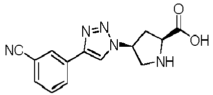 | (2*S*,4*S*)-4-(4-(3-cyanophenyl)-1*H*-1,2,3-triazol-1-yl)pyrrolidine-2-carboxylic acid | A | 5.309 | 284.1 |
| 22 | 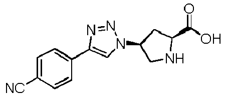 | (2*S*,4*S*)-4-(4-(4-cyanophenyl)-1*H*-1,2,3-triazol-1-yl)pyrrolidine-2-carboxylic acid | A | 5.371 | 284.1 |
| 23 | 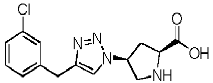 | (2*S*,4*S*)-4-(4-(3-chlorobenzyl)-1 *H*-1,2,3-triazol-1-yl)pyrrolidine-2-carboxylic acid | A | 6.487 | 307.0 |
| 24 | 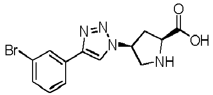 | (2*S*,4*S*)-4-(4-(3-bromophenyl)-1 *H*-1,2,3-triazol-1-yl)pyrrolidine-2-carboxylic acid | A | 6.733 | 339.0 |
| 25 | 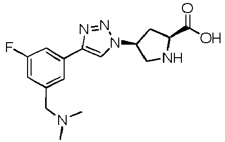 | (2*S*,4*S*)-4-(4-(3-((dimethylamino)methyl )-5-fluorophenyl)-1 *H-*1,2,3-triazol-1-yl)pyrrolidine-2-carboxylic acid | A | 2.429 | 334.2 |
| 26 | 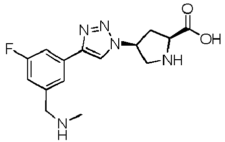 | (2*S*,4*S*)-4-(4-(3-fluoro-5-((methylamino)methyl)p henyl)-1*H*-1,2,3-triazol-1-yl)pyrrolidine-2-carboxylic acid | C | 1.606 | 320.2 |
| 27 | 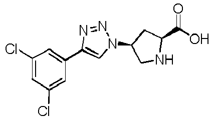 | (2*S*,4*S*)-4-(4-(3,5-dichlorophenyl)-1*H-*1,2,3-triazol-1-yl)pyrrolidine-2-carboxylic acid | A | 8.150 | 327.0 |
| 28 | 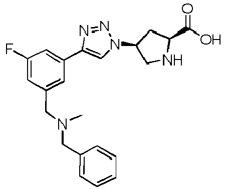 | (2*S*,4*S*)-4-(4-(3-((benzyl(methyl)amino) methyl)-5-fluorophenyl)-1*H*-1,2,3-triazol-1-yl)pyrrolidine-2-carboxylic acid | A | 5.268 | 410.2 |
| 29 | 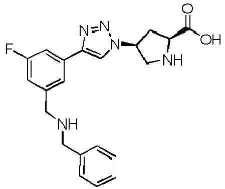 | (2*S*,4*S*)-4-(4-(3-((benzylamino)methyl)-5-fluorophenyl)-1*H-*1,2,3-triazol-1-yl)pyrrolidine-2-carboxylic acid | A | 4.790 | 307.1 |
| 30 | 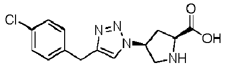 | (2*S*,4*S*)-4-(4-(4-chlorobenzyl)-1 H-1,2,3-triazol-1-yl)pyrrolidine-2-carboxylic acid | A | 6.841 | 307.0 |

### BIOLOGICAL ACTIVITY

### Pharmacological study

### Human α₂δ-1 subunit of Caᵥ2.2 calcium channel assay

Human α₂δ-1 enriched membranes (2.5 µg) were incubated with 15 nM of radiolabeled [3H]-Gabapentin in assay buffer containing Hepes-KOH 10mM, pH 7.4. NSB (non specific binding) was measured by adding 10 µM pregabalin. The binding of the tested compound was measured at either one concentration (% inhibition at 1 or 10 µM) or five different concentrations to determine affinity values (Ki). After 60 min incubation at 27 °C, binding reaction was terminated by filtering through Multiscreen GF/C (Millipore) presoaked in 0.5 % polyethyleneimine in Vacuum Manifold Station, followed by 3 washes with ice-cold filtration buffer containing 50 mM Tris-HCI, pH 7.4. Filter plates were dried at 60 °C for 1 h and 30 µL of scintillation cocktail were added to each well before radioactivity reading. Readings were performed in a Trilux 1450 Microbeta radioactive counter (Perkin Elmer).

### Results:

As this invention is aimed at providing compounds which act as ligands of the α₂δ subunit of voltage-gated calcium channels and especially compounds which have a binding expressed as Kᵢ responding to the following scales:
Kᵢ(α₂δ-1) is preferably < 10000 nM, more preferably < 5000 nM, or even more preferably < 500 nM.

The following scale has been adopted for representing the binding to the α₂δ-1 subunit of voltage-gated calcium channels expressed as Kᵢ:

| | |
|---|---|
| + | Kᵢ(α₂δ-1) >= 5000 nM |
| ++ | 500 nM <= Kᵢ(α₂δ-1) <5000 nM |
| +++ | Kᵢ(α₂δ-1) <500 nM |

Preferably, when Kᵢ(α₂δ-1) > 5000 nM, the following scale has been adopted for representing the binding to the α₂δ-1 subunit of voltage-gated calcium channels:

| | |
|---|---|
| + | Kᵢ(α₂δ-1) > 5000 nM or inhibition ranges between 1 % and 50 % |

All compounds prepared in the present application exhibit binding to the α₂δ-1 subunit of voltage-gated calcium channels, in particular the following binding results are shown:

| **EXAMPLE** | **α₂δ-1 Ki (nM)** |
|---|---|
| 1 | +++ |
| 2 | +++ |
| 3 | + |
| 4 | + |
| 5 | ++ |
| 6 | + |
| 7 | ++ |
| 8 | + |
| 9 | ++ |
| 10 | +++ |
| 11 | + |
| 12 | ++ |
| 13 | + |
| 14 | ++ |
| 15 | + |
| 16 | + |
| 17 | ++ |
| 18 | + |
| 19 | + |
| 20 | + |
| 21 | + |
| 22 | + |
| 23 | + |
| 24 | ++ |
| 25 | + |
| 26 | + |
| 27 | + |
| 28 | + |
| 29 | + |
| 30 | + |

## Claims

1. Compound of general formula (I): 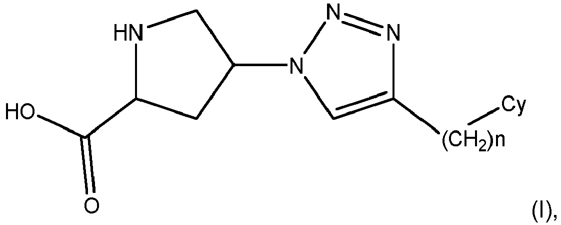 wherein,
Cy represents a group selected from: 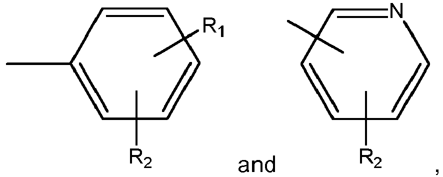
n is 0 or 1;
R₁ is selected from the group consisting of hydrogen, halogen, -CN, linear or branched substituted or unsubstituted C₁₋₆ alkyl, linear or branched substituted or unsubstituted C₂₋₆ alkenyl, and linear or branched substituted or unsubstituted C₁₋₆ alkoxy;
R₂ is selected from the group consisting of hydrogen, halogen, linear or branched substituted or unsubstituted C₁₋₆ alkyl, linear or branched substituted or unsubstituted C₂₋₆ alkenyl, linear or branched substituted or unsubstituted C₁₋₆ alkoxy and -(CH₂)ₚ-NRₐR_{b},
p is 0, 1, 2, or 3;
Rₐ and R_{b} are independently selected from hydrogen, linear or branched C₁₋₆ alkyl and unsubstituted or unsubstituted benzyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding physiologically acceptable salt or solvate thereof;
wherein the following compounds are excluded:
• 4-(4-(3-chlorophenyl)-1*H*-1,2,3-triazol-1-yl)pyrrolidine-2-carboxylic acid,
• 4-(4-(2-methoxyphenyl)-1*H*-1,2,3-triazol-1-yl)pyrrolidine-2-carboxylic acid,
• 4-(4-(3-methoxyphenyl)-1*H*-1,2,3-triazol-1-yl)pyrrolidine-2-carboxylic acid,
• 4-(4-(4-fluorophenyl)-1*H*-1,2,3-triazol-1-yl)pyrrolidine-2-carboxylic acid,
• 4-(4-(4-methoxyphenyl)-1*H*-1,2,3-triazol-1-yl)pyrrolidine-2-carboxylic acid,
• 4-(4-(pyridin-2-yl)-1*H*-1,2,3-triazol-1-yl)pyrrolidine-2-carboxylic acid,
• 4-(4-phenyl-1*H*-1,2,3-triazol-1-yl)pyrrolidine-2-carboxylic acid,
• 4-(4-(2-chloro)-1*H*-1,2,3-triazol-1-yl)pyrrolidine-2-carboxylic acid,
• 4-(4-(3,5-difluorophenyl)-1*H*-1,2,3-triazol-1-yl)pyrrolidine-2-carboxylic acid.

2. A compound according to claim 1, wherein Cy represents a group:

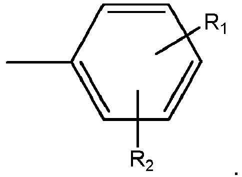

3. A compound according to claim 2, wherein R₁ represents a hydrogen, a halogen, more preferably a Br, Cl, or F, a -CN, an unsubstituted C₁₋₆ alkyl, preferably a methyl (-CH₃), or a unsubstituted C₁₋₆ alkoxy, preferably a methoxy (-O-CH₃).

4. A compound according to any of claims 2 to 3, wherein R₂ represents a hydrogen, a halogen, more preferably a Br, Cl, or F, -(CH₂)ₚ-NRₐR_{b}, wherein p is 1, Rₐ is a hydrogen or an unsubstituted C₁₋₆ alkyl, preferably a methyl (-CH₃), and R_{b} is a hydrogen, an unsubstituted C₁₋₆ alkyl, preferably a methyl (-CH₃), or an unsubstituted benzyl.

5. A compound according to ny of claims 2 to 4, wherein Cy represents a group:

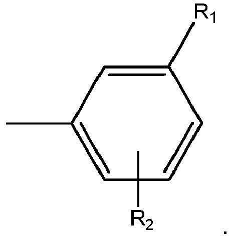

6. A compound according to claim 1, wherein Cy represents a group:

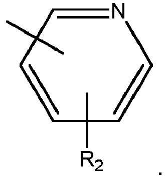

7. A compound according to claim 6, wherein R₂ represents a hydrogen or a halogen, more preferably a Cl.

8. A compound according to any of claims 6 to 7, where Cy represents a group:

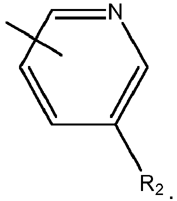

9. A compound according to any of claims 1 to 8 selected form the group consisiting of:
• (2*S*,4*S*)-4-(4-(3-fluorophenyl)-1*H*-1,2,3-triazol-1-yl)pyrrolidine-2-carboxylic acid,
• (2*S*,4*S*)-4-(4-(*m*-tolyl)-1*H*-1,2,3-triazol-1-yl)pyrrolidine-2-carboxylic acid,
• (2*S*,4*S*)-4-(4-(3,4-difluorophenyl)-1*H*-1,2,3-triazol-1-yl)pyrrolidine-2-carboxylic acid,
• (2*S*,4*S*)-4-(4-(2-chloro-4-methoxyphenyl)-1*H*-1,2,3-triazol-1-yl)pyrrolidine-2-carboxylic acid,
• (2*S*,4*S*)-4-(4-(2,4-dichlorophenyl)-1*H*-1,2,3-triazol-1-yl)pyrrolidine-2-carboxylic acid,
• (2*S*,4*S*)-4-(4-(3,4-dichlorophenyl)-1*H*-1,2,3-triazol-1-yl)pyrrolidine-2-carboxylic acid,
• (2*S*,4*S*)-4-(4-benzyl-1*H*-1,2,3-triazol-1-yl)pyrrolidine-2-carboxylic acid,
• (2*S*,4*S*)-4-(4-(4-bromophenyl)-1*H*-1,2,3-triazol-1-yl)pyrrolidine-2-carboxylic acid,
• (2*S*,4*S*)-4-(4-(pyridin-3-yl)-1*H*-1,2,3-triazol-1-yl)pyrrolidine-2-carboxylic acid,
• (2*S*,4*S*)-4-(4-(pyridin-4-yl)-1*H*-1,2,3-triazol-1-yl)pyrrolidine-2-carboxylic acid,
• (2*S*,4*S*)-4-(4-(5-chloropyridin-3-yl)-1*H*-1,2,3-triazol-1-yl)pyrrolidine-2-carboxylic acid,
• (2*S*,4*S*)-4-(4-(3-cyanophenyl)-1*H*-1,2,3-triazol-1-yl)pyrrolidine-2-carboxylic acid,
• (2*S*,4*S*)-4-(4-(4-cyanophenyl)-1*H*-1,2,3-triazol-1-yl)pyrrolidine-2-carboxylic acid,
• (2*S*,4*S*)-4-(4-(3-chlorobenzyl)-1*H*-1,2,3-triazol-1-yl)pyrrolidine-2-carboxylic acid,
• (2*S*,4*S*)-4-(4-(3-bromophenyl)-1*H*-1,2,3-triazol-1-yl)pyrrolidine-2-carboxylic acid,
• (2*S*,4*S*)-4-(4-(3-((dimethylamino)methyl)-5-fluorophenyl)-1*H*-1,2,3-triazol-1 - yl)pyrrolidine-2-carboxylic acid,
• (2*S*,4*S*)-4-(4-(3-fluoro-5-((methylamino)methyl)phenyl)-1*H*-1,2,3-triazol-1-yl)pyrrolidine-2-carboxylic acid,
• (2*S*,4*S*)-4-(4-(3,5-dichlorophenyl)-1*H*-1,2,3-triazol-1-yl)pyrrolidine-2-carboxylic acid,
• (2*S*,4*S*)-4-(4-(3-((benzyl(methyl)amino)methyl)-5-fluorophenyl)-1*H*-1,2,3-triazol-1-yl)pyrrolidine-2-carboxylic acid,
• (2*S*,4*S*)-4-(4-(3-((benzylamino)methyl)-5-fluorophenyl)-1*H*-1,2,3-triazol-1 - yl)pyrrolidine-2-carboxylic acid, and
• (2*S*,4*S*)-4-(4-(4-chlorobenzyl)-1*H*-1,2,3-triazol-1-yl)pyrrolidine-2-carboxylic acid.

10. A process for the preparation of a compound of formula (I) according to claim 1 comprising the steps of:
• reacting a stock solution of a compound of formula (II): 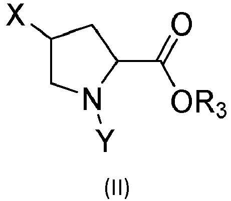 wherein X represents a leaving group, R₃ represents an C₁₋₆alkyl group, Y represents a protecting group of the amino function
• with an azide reagent of formula (III):
N₃Z (III),
wherein Z is selected from sodium, potassium, tosyl, and trimethylsilyl, in an organic solvent,
• pumpimg the resulting solution under continuous flow through a first reactor at a temperature of 50 to 200 °C and pressure of 0 to 750 psi,
• mixing continuously the resulting mixture with a stock solution of a compound of formula (IV): 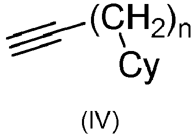 wherein Cy and n are as defined in claim 1,
in an organic solvent,
• pumping the resulting mixture under continuous flow through a second reactor at a temperature of 0 to 300 °C, and a pressure of 0 to 1000 psi, to give a compound of formula (V): 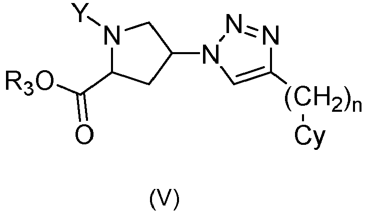
• which is reacted with an acid, in an appropriate solvent, optionally in the presence of an additive,
to give a compound of formula (I) according to claim 1.

11. A compound of formula (I) below for use as a medicament: 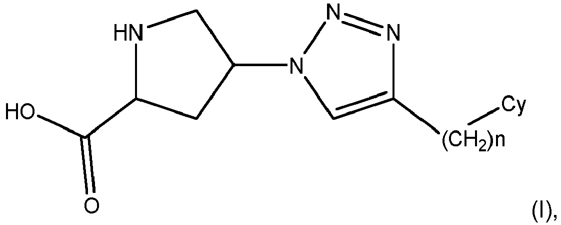 wherein,
Cy represents a group selected from: 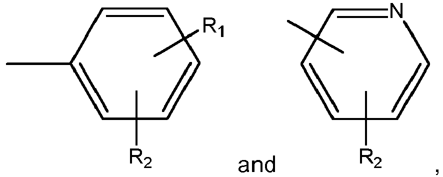
n is 0 or 1;
R₁ is selected from the group consisting of hydrogen, halogen, -CN, linear or branched substituted or unsubstituted C₁₋₆ alkyl, linear or branched substituted or unsubstituted C₂₋₆ alkenyl, and linear or branched substituted or unsubstituted C₁₋₆ alkoxy;
R₂ is selected from the group consisting of hydrogen, halogen, linear or branched substituted or unsubstituted C₁₋₆ alkyl, linear or branched substituted or unsubstituted C₂₋₆ alkenyl, linear or branched substituted or unsubstituted C₁₋₆ alkoxy and -(CH₂)ₚ-NRₐR_{b},
p is 0, 1, 2, or 3;
Rₐ and R_{b} are independently selected from hydrogen, linear or branched C₁₋₆ alkyl and unsubstituted or unsubstituted benzyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding physiologically acceptable salt or solvate thereof.

12. A compound of formula (I) according to claim 11 for use in the treatment of pain.

13. A compound of formula (I) according to claim 12 for use in the treatment of a disease or condition selected from medium to severe pain, visceral pain, chronic pain, cancer pain, migraine, inflammatory pain, acute pain or neuropathic pain, allodynia, hyperalgesia, mechanical allodynia and thermal hyperalgesia.

14. A pharmaceutical composition which comprises a compound of formula (I) according to claim 11 or a pharmaceutically acceptable salt or stereoisomer thereof, and a pharmaceutically acceptable carrier, adjuvant or vehicle.
